# EUROPEAN PATENT SPECIFICATION

(11) **EP 4 633 372 B1**
(45) Date of publication and mention of the grant of the patent: **11.11.2026**
(21) Application number: 23828417.8
(22) Date of filing: 12.12.2023
(51) Int. Cl.: A01N 63/32, A01P 1/00, A01P 3/00, C05F 11/08, A01P 21/00

(54) **A PLANT BIOSTIMULANT AND/OR BIOCONTROL COMPOSITION FOR IMPROVING PLANT GROWTH**
PFLANZENBIOSTIMULANS UND/ODER BIOKONTROLLZUSAMMENSETZUNG ZUR VERBESSERUNG DES PFLANZENWACHSTUMS
BIOSTIMULANT VÉGÉTAL ET/OU COMPOSITION DE LUTTE BIOLOGIQUE POUR AMÉLIORER LA CROISSANCE DES PLANTES

(30) Priority: 12.12.2022 FI 20226096
(43) Date of publication of application: 22.10.2025
(73) Proprietor: Neova Oy, 40100 Jyväskylä (FI)
(72) Inventor: HAKALAHTI, Teija, 40100 Jyväskylä (FI); HÄMÄLÄINEN, Katja, 40100 Jyväskylä (FI); HYTÖNEN, Kaisa, 40100 Jyväskylä (FI)
(74) Representative: Laine IP Oy
(86) International application number: PCT/FI2023/050687
(87) International publication number: WO 2024/126897

(56) References cited:
- WO-A2-2012/037352
- FILIPOWICZ NATALIA ET AL: "Determination of phenol biodegradation pathways in three psychrotolerant yeasts, Candida subhashii A011, Candida oregonensis B021 and Schizoblastosporion starkeyi-henricii L012, isolated from Rucianka peatland", ENZYME AND MICROBIAL TECHNOLOGY, STONEHAM, MA, US, vol. 141, 6 September 2020 (2020-09-06), XP086289178, ISSN: 0141-0229, [retrieved on 20200906], DOI: 10.1016/J.ENZMICTEC.2020.109663
- FILIPOWICZ NATALIA ET AL: "Isolation and Characterization of Phenol-Degrading Psychrotolerant Yeasts", WATER, AIR, SPRINGER INTERNATIONAL PUBLISHING, CHAM, vol. 228, no. 6, 22 May 2017 (2017-05-22), pages 1 - 16, XP036255222, ISSN: 0049-6979, [retrieved on 20170522], DOI: 10.1007/S11270-017-3391-8
- GOLUBEV W I ET AL: "A study ofSchizoblastosporion starkeyi-henriciiisolates from northern and southern hemispheres of the Earth", MICROBIOLOGY, CONSULTANTS BUREAU, NEW YORK, NY, US, vol. 83, no. 5, 25 September 2014 (2014-09-25), pages 661 - 665, XP035397680, ISSN: 0026-2617, [retrieved on 20140925], DOI: 10.1134/S0026261714050117
- HAJ-AHMAD Y ET AL: "THIAMINE SECRETION IN YEAST", CANADIAN JOURNAL OF MICROBIOLOGY, NRC RESEARCH PRESS, CA, vol. 38, no. 11, 1 January 1992 (1992-01-01), pages 1156 - 1161, XP009034508, ISSN: 0008-4166

## Description

### FIELD

The present disclosure relates to the field of plant-microorganism interactions. In particular, the present disclosure relates to methods and compositions for increasing plant growth characteristics and/or plant disease resistance by growing the plant in the presence of plant growth-promoting microbial inoculants, or in the presence of compositions comprising the microorganisms described herein or metabolites thereof.

### BACKGROUND

In order to enhance plant and crop production and overcome poor soil quality, the use of fertilizers and pesticides is widespread and well-known. Commercially available fertilizers typically comprise inorganic chemical fertilizers. Such fertilizers are often associated with environmentally damaging consequences, such as eutrophication of water bodies. Moreover, as it is now widely accepted that pesticides have a major effect on biological diversity, the need for environmentally safe plant pathogen control is also evident in agriculture. Consequently, there has been a growing interest in the field to improve crop yield using ecofriendly and more sustainable approaches. A promising practice is the use of microbial based biostimulants, such as soil microbes or plant symbiont microbes, that enhance plant growth or performance, increase tolerance to abiotic stress, plant diseases and/or improve the resource use efficiency.

Plant growth promotion is a complex phenomenon rarely attributable to a single mechanism. Soil bacteria and fungi that associate with plant roots and have a positive effect on plant growth usually improve the growing environment for the plant at least by fixing nitrogen, producing plant hormones or antibiotics, solubilizing soil phosphates or iron, and/or inhibiting pathogenic microorganisms. Accordingly, microbes, such as bacteria and fungi, have been commercially used as biostimulants or as biocontrol agents. Yeasts, such as *Saccaromyces cerevisiae* are used in such preparations either in active or inactive form (metabolites). In the case of fungi, at least *Trichoderma* sp. have been utilized for biological control of soil-borne pathogens.

WO2012037352 discloses a composition for reducing pathogens in soil and improving plant growth comprising intracellular components of lysed yeast cells. Among the beneficial yeasts, *Nadsonia* and *Schizoblastosporion* are mentioned.

Filipowicz et al., 2020, Filipowicz et al., 2017 as well as Golubev et al., 2014, disclose isolated *Schizoblastosporion starkeyi-henricii* strains.

Haj-Ahmad et al., 1992, disclose presence of thiamine in yeast cultures of genus *Nadsonia.*

With increasing demands for food and energy as the world's human population expands, higher crop yields are needed for both food and biofuel production. Due to climate change crop plants are also more frequently exposed to both abiotic and biotic stresses that have negative impact on plant performance. Thus, there still exists a need for identifying and isolating additional microorganisms that promote growth of crop plants and could be utilized commercially as biostimulants or biocontrol agents.

### SUMMARY OF THE INVENTION

The invention is defined by the features of the independent claims. Some specific embodiments are defined in the dependent claims.

According to a first aspect of the present invention, there is provided a plant biostimulant and/or biocontrol composition for improving plant growth or plant development comprising a yeast of genus *Nadsonia* or killed and/or lysed cells of the yeast, wherein said yeast is the yeast strain of *Nadsonia starkeyi-henricii* deposited at the VTT Culture Collection with accession number VTT C-221063.

According to a second aspect of the present invention, there is provided a fertilizer comprising the plant biostimulant composition comprising a yeast of genus *Nadsonia* or killed and/or lysed cells of the yeast, wherein said yeast is the yeast strain of *Nadsonia starkeyi-henricii* deposited at the VTT Culture Collection with accession number VTT C-221063.

According to a third aspect of the present invention, there is provided a *Nadsonia starkeyi-henricii* yeast strain deposited at VTT Culture Collection with accession number VTT C-221063.

According to a fourth aspect of the present invention, there is provided a method for improving plant growth or plant development comprising a step of applying to a plant, seed thereof, or soil where the plant is cultivated a plant biostimulant composition comprising a yeast of genus *Nadsonia* or killed and/or lysed cells of the yeast, wherein said yeast is the yeast strain of *Nadsonia starkeyi-henricii* deposited at the VTT Culture Collection with accession number VTT C-221063. According to a fifth aspect of the present invention, there is provided a method for improving soil quality or remediating degraded soil, the method comprising applying to the soil, or to the plants or plant seeds in said soil, an effective amount of a fertilizer comprising the plant biostimulant composition comprising a yeast of genus *Nadsonia* or killed and/or lysed cells of the yeast, wherein said yeast is the yeast strain of *Nadsonia starkeyi-henricii* deposited at the VTT Culture Collection with accession number VTT C-221063.

### BRIEF DESCRIPTION OF THE DRAWINGS

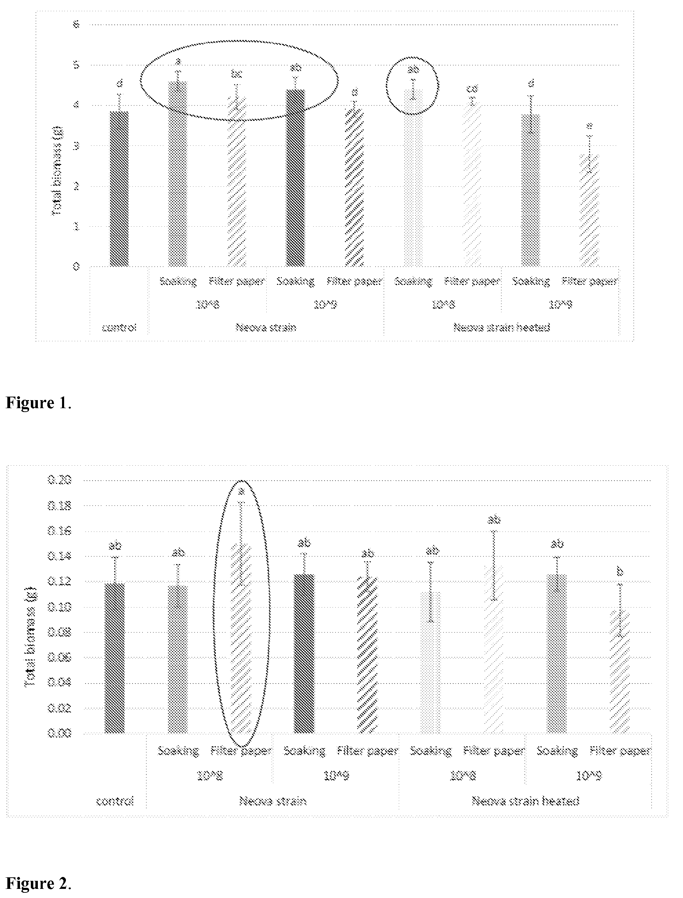

FIGURE 1. The growth-promoting effect of a novel yeast strain ("Neova strain") and its metabolites (heated samples) on maize. Maize seeds were treated with 10⁸ or 10⁹ yeast - solutions by soaking the seeds or by wetting the filter paper with either yeast product. Maize total biomass (g) of each treatment and control is shown. Data presented are the mean ± standard deviation for the 6 replicates. Letters correspond to homogenous group after LSD statistical analysis (α=0.05).
FIGURE 2. The growth-promoting effect of a novel yeast strain ("Neova strain") and its metabolites (heated samples) on dwarf tomato. Dwarf tomato seeds were treated with 10⁸ or 10⁹ yeast solutions by soaking the seeds or by wetting the filter paper with either yeast product. Dwarf tomato total biomass (g) of each treatment and control is shown. Data presented are the mean ± standard deviation for the 6 replicates. Letters correspond to homogenous group after LSD statistical analysis (α=0.05).

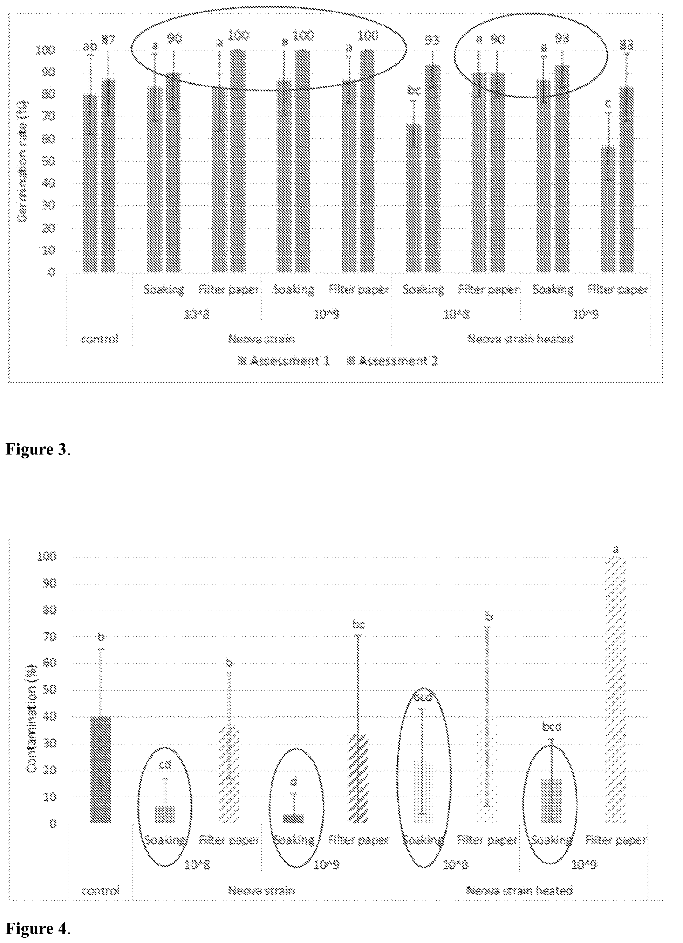

FIGURE 3. Dwarf tomato germination rate (%). For details see Example 2. Data presented are the mean ± standard deviation for the 6 replicates. Letters correspond to homogenous group after LSD statistical analysis (α=0.05).
FIGURE 4. The biocontrol effect of a novel yeast strain (Neova strain) and its metabolites (heated samples) against *Fusarium graminearum* infection on wheat. Wheat seeds were treated with 10⁸ or 10⁹ yeast cells/ml solutions by soaking the seeds or by wetting the filter paper with either of the yeast products and the seeds were infected with *Fusarium graminearum.* The figure shows *Fusarium* contamination rate (%) of each treatment and control. Data presented are the mean ± standard deviation for the 6 replicates. Letters correspond to homogenous group after LSD statistical analysis (α=0.05).

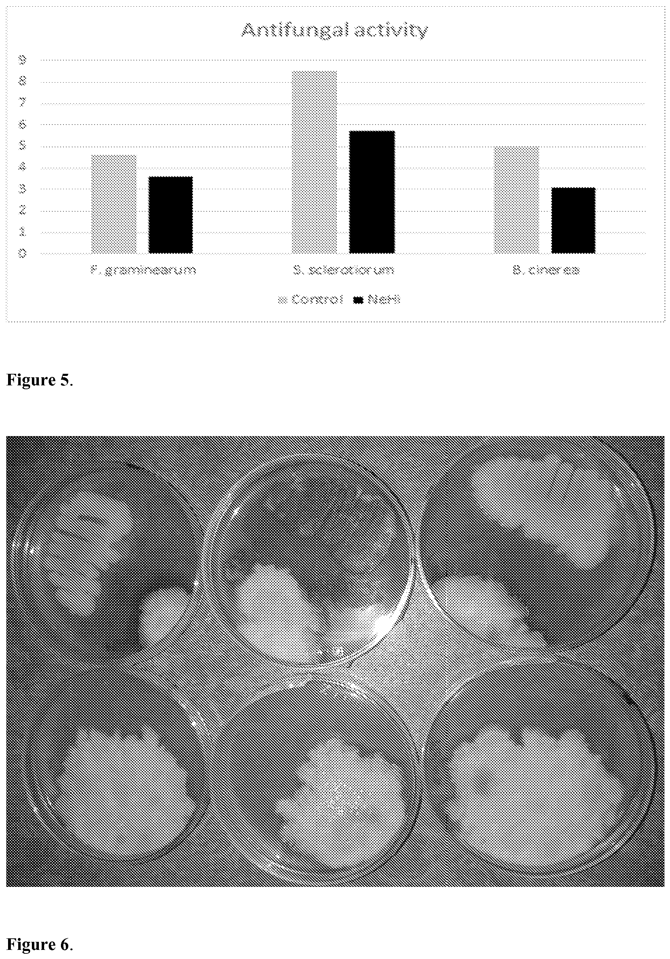

FIGURE 5. Antifungal activity of living Neova strain (NeHi). Bars indicates the growth (cm) of *F. graminearum, S. sclerotiorum* and *B. cinerea* on PDA plates with the living yeast strain (black bars) or in control plates (grey bars).
FIGURE 6. Neova strain (NeHi) clearly reduced the growth of *M. fructicola.* Upper row; the growth of *M. fructicola* and yeast NeHi on PDA plate after two months from the beginning of the experiment. Lower row; the growth of *M. fructicola* on PDA plates after two months from the beginning of the experiment.

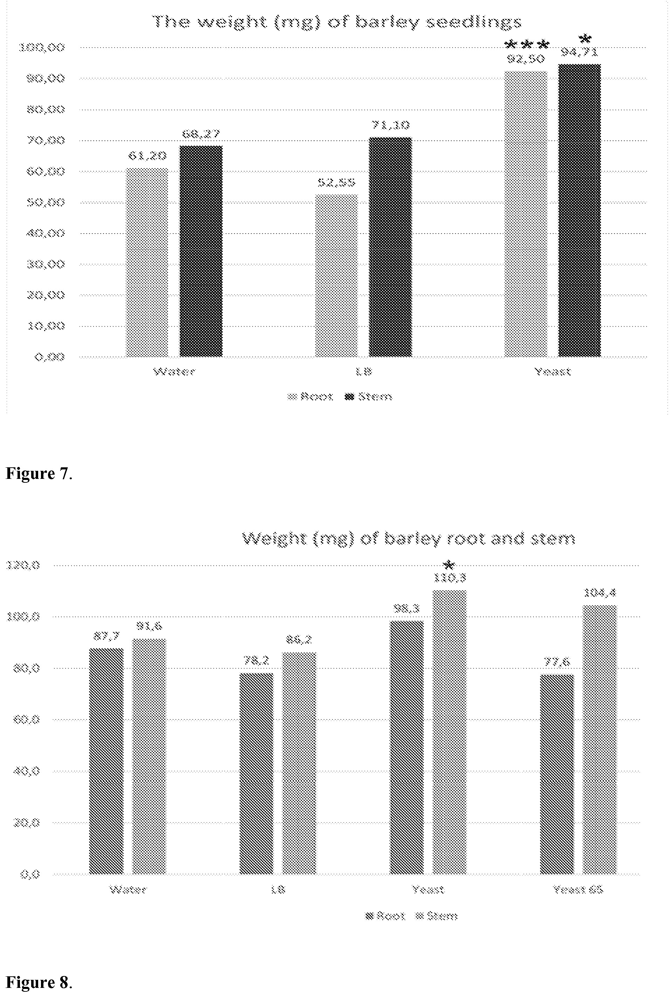

FIGURE 7. Weight of barley root and stem when barley seeds were inoculated with Neova strain (Yeast). Treatments were compared to water treatment control. *significance less than 0.05; **significance less than 0.01; ***significance less than 0.001.
FIGURE 8. Weight of barley root and stem when barley seeds were inoculated with Neova strain (Yeast) or its metabolites (heat-treated yeast suspension, Yeast 65). Treatments were compared to water treatment control.

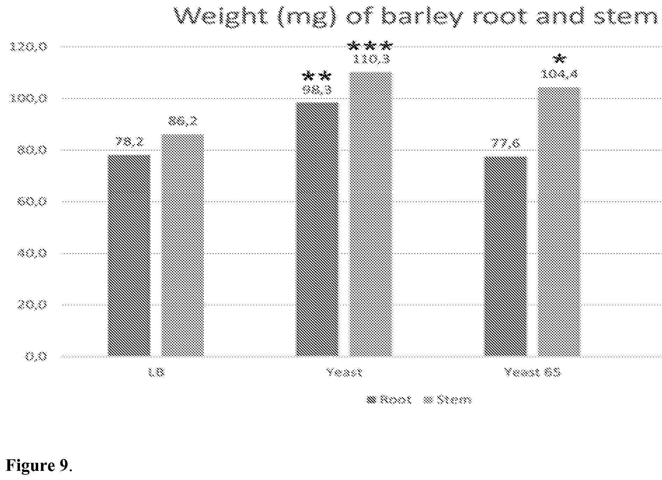

FIGURE 9. Weight of barley root and stem when barley seeds were inoculated with Neova strain (Yeast) or its metabolites (heat-treated yeast suspension, Yeast 65). Treatments were compared to LB treatment control.

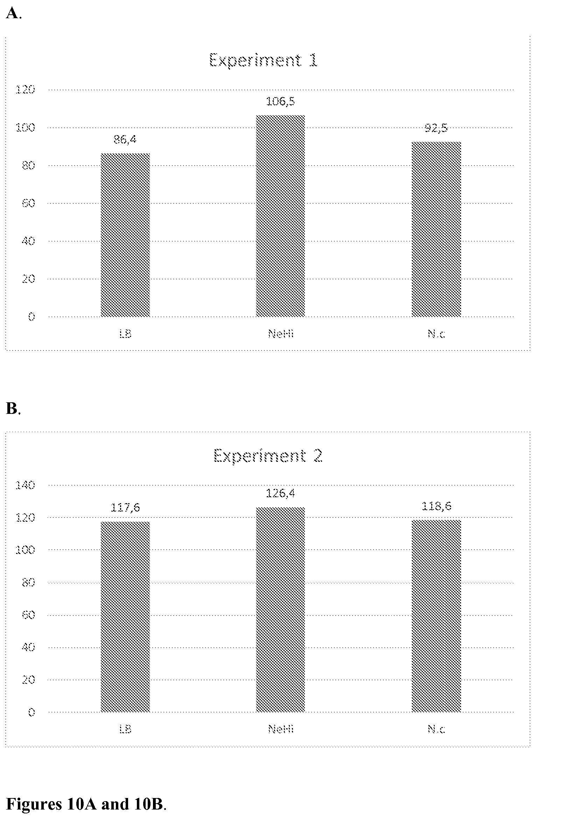

FIGURE 10. The effect of the Neova strain (NeHi) yeast on barley. (A) In the first experiment, Neova yeast increased the growth of barley roots by 23.3 % and *Nadsonia commutata* 7.1 % when compared to LB treatment. (B) In the second experiment, Neova yeast increased the growth of barley roots by 7.5 % and *N. commutata* 0.9 % when compared to LB treatment.

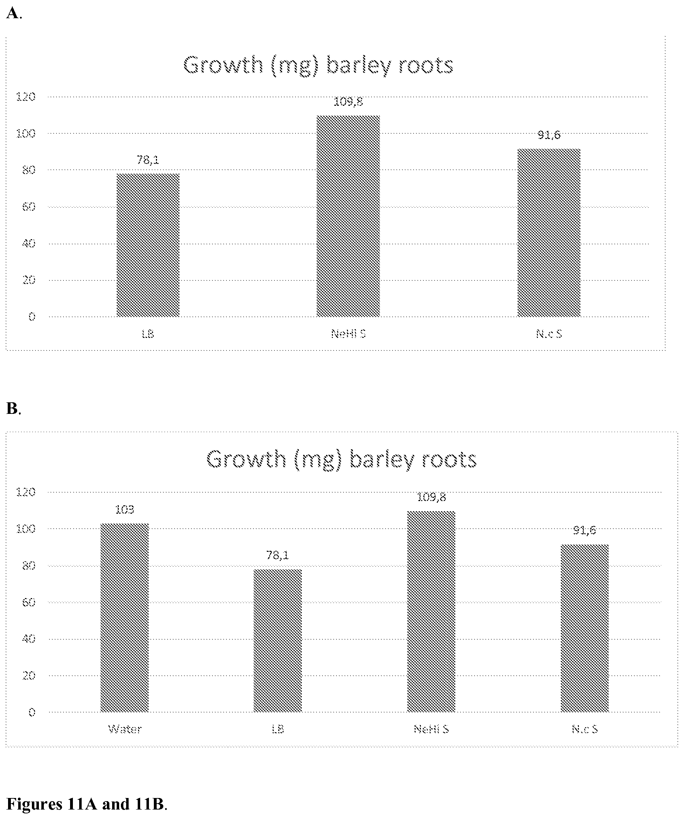

FIGURE 11. The effect of the Neova strain (NeHi) yeast on barley, when the yeast cells have been separated from the growing medium. (A) Neova yeast (cells suspended in water) increased the growth of barley roots by 40.6 % and *N. commutata* 17.3 % when compared to LB treatment. (B) Neova yeast increased the growth of barley roots by 6.6 % when compared to water and LB treatment and *N. commutata* increased the growth of barley roots when compared to LB treatment. LB, Luria broth; NeHi S, Neova yeast grown in LB medium for 40 hours at room temperature, growing medium was subsequently centrifugated and cells suspended in water; N.c *S, Nadsonia commutata* grown in LB medium for 40 hours at room temperature, growing medium was subsequently centrifugated and cells suspended in water.

### EMBODIMENTS

In the present context, the term "plant growth" refers to the growth of any plant part, including stems, leaves, flowers, inflorescences, fruits, and roots. Growth may refer to the rate of growth of any one of these plant parts. The term "plant development" as understood herein is the process by which structures originate and mature as a plant grows. Plants produce new tissues and structures throughout their life from clusters of undifferentiated cells called meristems.

In the present context, the term "biocontrol" refers to a method of controlling plant diseases caused by bacteria or fungi using other bacteria and fungi capable of controlling plant diseases, preferably the yeast strain presented herein. Biocontrol relies on natural mechanisms, but typically also involves an active human management role. The present disclosure specifically discloses biopesticides, i.e. types of pesticides derived from such natural organisms as microbes, particularly from the yeast strain presented herein. The term "biostimulant" refers to a substance or microorganism applied to plants with the aim to enhance nutrition efficiency, abiotic stress tolerance and/or crop quality traits, regardless of its nutrient content.

The term "fungicide or antifungal" means the capacity of a substance to increase the mortality or inhibit the growth rate of fungi.

The term "metabolite" refers herein to any compound, substance or by-product of a yeast metabolism or fermentation having plant growth promoting activity or biopesticide/biocontrol activity.

The meaning of "improving soil quality" includes at least increasing the amount and/or availability of nutrients, bioactive compounds, and/or micro-organisms required by or beneficial to plants in soil. Thus, improving soil quality by use of microbial products and fertilizer compositions of the present disclosure thereby assists and promotes the growth of plants in the soil.

In accordance with the present disclosure, a novel yeast, a plant biostimulant and/or biocontrol (e.g. antimicrobial, preferably fungicidal) composition and a fertilizer composition comprising said yeast are presented which find application in increasing plant productivity and improving soil quality.

In an embodiment, the yeast used in the invention is the strain deposited by the applicant on the 8^{th} of November of 2022, at the VTT Culture Collection, VTT Technical Research Centre of Finland Ltd, Tietotie 2, Espoo P.O. Box 1000, FI-02044 VTT, Finland. The deposit of the deposited strain designated here as a strain of species *Nadsonia starkeyi-henricii* (i.e. "Neova yeast/strain" or "NeHi") was given the accession number VTT C-221063 once said International Depositary Authority (i.e. the VTT Culture Collection) declared that said strain in question was viable, under the stipulations of the Budapest Treaty on the International Recognition of the Deposit of Microorganisms for the Purpose of Patent Procedure.

The concentrations of the yeast strain to be added to the fertilizer/biostimulant compositions as disclosed herein will depend on a variety of factors including the identity of the yeast employed, the plant species being treated, the nature and condition of the soil to be treated, the exact nature of the fertilizer/biostimulant composition to be applied, the form in which the fertilizer/biostimulant is applied and the means by which it is applied, and the stage of the plant growing season during which application takes place. For any given case, appropriate concentrations may be determined by one of ordinary skill in the art using routine experimentation. By way of example only, the concentration of each strain present in the inoculant or fertilizer composition may be from about 1 × 10² cfu/ml to about 1 × 10¹⁰ cfu/ml.

Novel Neova yeast as described herein was identified by the methods described by Marttinen et al (2020). Internal transcribed spacer (ITS) sequences were obtained by PCR using the standard ITS4 and ITS5 primers. The ITS sequence obtained for the novel Neova strain is the following:

TGGGTCATTAACGAGTTTTGTACCAATACGGTACAATCTTCATCCATAACACCTGTG
AACTTTACATTAACATTTGCTTTGGCTGGACGTAAAAAACCTAGCCAAAGGATTAAA
CAAAACCTTTTATTTAATAACCTATTGTCTGAATTGTAATAATTTTAATTATTCAAAA
CTTTCAACAACGGATCTCTTGGTTCTCGCATCGATGAAGAACGCAGCGAAATGCGAT
AAGTATTGTGAATTGCAGATTTGTGAATCATCGAATTTTTGAACGCACATTGCGCCC
TCTGGTATTCCGGGGGGCATGCCTGTTCGAGCGTCATTGCTTTCTCAAACCCTCTGGT
TTGGTGTTGAATTTATTATGTAGCAATACATATGATTCGAAAAGCATTGGCAAGGCT
ATTTGAGCTTTTCTTACAACGTATTAGGTTTTACCAACTCGTTGGCTACAGTAAGTTT
GAAAATAAAGCCTGGCTTTAGAACTCTCTAAAGTTTGACCGGATCAGGTAGGATTAC
CCGCTGAACTTAAGCATATCATAAAAGGGAAGGAAAAAAAAAAA (SEQ ID NO:1)

A BLAST search with the NCBI database was used to compare the obtained ITS sequence, including ITS1, 5.8S and ITS2 regions (~500-600 nt) with other fungal sequences. BLAST search at NCBI database showed sequence identity of 99.26 % with *Nadsonia starkeyhenricii* isolate (Query length 561 nucleotides, query coverage 98 %).

For the purposes of the present disclosure, the yeast as described herein can be cultured in any of the growth media known in the field as suitable for culturing yeasts. Examples of the culture medium as used herein can include ingredients such as peptone, soy peptone, molasses, potato starch, yeast extract powder, or combinations thereof.

To aid in survival of microorganisms present in the fertilizer/biostimulant compositions as disclosed herein upon application in the environment, the yeast may be encapsulated in, for example, a suitable polymeric matrix. Encapsulation may also provide extended-release and/or slow-release properties for the composition. Those skilled in the art will appreciate that any suitable encapsulation material or matrix may be used. For instance, solid form fertilizer particles can be encapsulated in water soluble coatings (for example dyed or undyed gelatin spheres or capsules), extended release coatings, or by micro-encapsulation to a free flowing powder using, for example, gelatin, polyvinyl alcohol, or ethylcellulose.

Those skilled in the art will appreciate that any plant may benefit from the application of the fertilizer/biostimulant compositions as disclosed herein to soil, seeds and/or vegetation. Particular embodiments are employed to aid the growth, development, yield or productivity of crops or other plants of economic value, including ornamentals and plants grown for oils or biofuel. The crop plant may be, for example, a food crop (for humans or other animals) such as any fruit, vegetable, nut, seed or grain producing plant. Exemplary crop plants include, but are not limited to, tubers and other below-ground vegetables (such as potatoes, beetroots, radishes, carrots, onions, etc.), cereals (such as rice, maize, wheat, barley, rye etc.), leaf vegetables (such as lettuces, chard, spinach etc.), other vegetables (such as tomatoes).

The fertilizer/biostimulant compositions as disclosed herein may be applied directly to plants, plant parts (such as foliage) or seeds, or alternatively may be applied to soil in which the plants are growing or to be grown or in which seeds have been or are to be sown. Application may be by any suitable means and may be on any suitable scale. For example, application may comprise pouring, spreading or spraying, including broad scale or bulk spreading or spraying, soaking of seeds before planting, and/or drenching of seeds after planting or seedlings. Those skilled in the art will appreciate that multiple means of application may be used in combination (for example soaking of seeds prior to planting followed by drenching of planted seeds and/or application to seedlings or mature plants). Seeds, seedlings or mature plants may be treated as many times as appropriate. The number of applications required can readily be determined by those skilled in the art depending on, for example, the plant in question, the stage of development of the plant at which treatment is initiated, the state of health of the plant, the growth, environmental and/or climatic conditions in which the plant is grown and the purpose for which the plant is grown. For example, in the case of flowering crops such as tomatoes, it may be desirable to apply the fertilizer/biostimulant compositions as disclosed herein once or more than once during the flowering period.

In accordance with the present disclosure, the fertilizer/biostimulant compositions as disclosed herein may be prepared in any suitable form depending on the means by which the fertilizer/biostimulant composition is to be applied to the soil or to plant seeds or vegetation. Suitable forms can include, for example, slurries, liquids, and solid forms. Solid forms include powders, granules, larger particulate forms and pellets. Liquids may include aqueous solutions and aqueous suspensions, and emulsifiable concentrates.

Additional components, such as components suitable for use as carriers of the other fertilizer components, may be incorporated into fertilizer/biostimulant compositions of the present disclosure, such as other micro-organisms, humic substances, sphagnum moss (*Sphagnum* spp.), peat, trace elements, organic and inorganic material, penetrants, macronutrients, micronutrients and other soil and/or plant additives.

Humus or humic substances that may be incorporated may include, but are not limited to, humic acid derived from, for example compost, peat, lignite or leonardite, fulvic acid and humates such as potassium humate.

Unless defined otherwise, all technical and scientific terms used herein have the same meaning as commonly understood by those of ordinary skill in the art to which the disclosure belongs. Although any methods and materials similar or equivalent to those described herein can be used in the practice or testing of the present disclosure, typical methods and materials are described.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

### EXPERIMENTAL SECTION

EXAMPLE 1. Investigating yeast and yeast metabolite growth-promoting effect to *Arabidopsis thaliana* fresh roots and fresh aerial biomass, in vitro.

Procedure: The trial was performed in Petri dishes in sterile conditions. *Arabidopsis* seeds were sown on growing medium. After 7 days, the most homogenous plantlets were transplanted on the same growing medium. Products were applied with a pipette (50µl/plant) on the whole plant 11 days after sowing. The trial was organized in 3 independent replicates of 1 Petri dish per modality. Each Petri dish contained 8 plantlets. A novel yeast strain, i.e. Neova strain, was compared to two control modalities grown without the presence of the yeast: a negative control treated with water and a positive control treated with a nutrient solution. The isolated strain was applied at ratio (2.88 × 10⁶ cells/ml × 0.041 ml (0.01%/ 24.3% DM) = 118 080 cells in 100 mL of water. The fresh aerial biomass and root biomass was measured at the end of the test (21 days from sowing).

Result: Neova yeast showed a positive impact on both aerial and root biomasses (Table 1). The growth-promoting effect was even statistically stronger than the positive control (groups a and b, respectively).

**Table 1. Neova yeast showed a positive impact on both aerial and root biomasses compared to controls, see Example 1. Data presented are the mean ± standard deviation for the 3 replicates. Letters correspond to homogenous group after LSD statistical analysis (α=0.05).**

| **Products** | **Fresh aerial biomass (mg)** | | **Fresh root biomass (mg)** | | **Total biomass (mg)** | |
|---|---|---|---|---|---|---|
| Optimal nutrient solution | 25.54 ± 1.49 | b | 4.65 ± 0.34 | bc | 30.18 ± 1.82 | b |
| Low nutrient solution = water | 20.53 ± 1.66 | c | 3.32 ± 0.19 | cd | 23.84 ± 1.85 | c |
| Neova yeast | 39.56 ± 3.42 | ab | 12.56 ± 2.1 | ab | 52.12 ± 5.4 | ab |

EXAMPLE 2. Investigating yeast and yeast metabolite growth-promoting effect to maize, wheat and dwarf tomato by treating the seeds with the products, in vitro.

Procedure: The trial was performed in Petri dishes on 2 different species: maize (variety FuturiX) and dwarf tomato (variety Azteck). Seeds were sanitized, rinsed and placed in Petri dishes (5 seeds/Petri dish) on a filter paper. The trial was performed using a Neova yeast culture and a heat-treated (65 °C for 10 min) suspension thereof. The described heat treatment kills and lyses yeast cells and thus the potential of the yeast strain metabolites can be evaluated. Samples were prepared by soaking the seeds or by wetting the filter paper (2ml/Petri dish) in/with a suspension. In sample preparation, the cell concentration of the cultured Neova yeast was 10⁸ and 10⁹ cells/ml. The trial was organized in 6 independent replicates of 1 Petri dish per modality. Each Petri dish contained 5 seeds. Evaluated modalities were compared to one control modality treated with water (2ml/Petri dish). Total biomass and germination percentage was measured after 10 days.

### Results

Maize total biomass (see Figure 1) was increased statistically significantly when maize seeds were treated with 10⁸ cells/ml yeast solutions by soaking the seeds and by wetting the filter paper with the product. Soaking seeds to a 10⁹ cells/ml yeast solution also increased total biomass statistically significantly. Heated Neova strain samples (yeast metabolites) also increased maize biomass when seeds were soaked to a 10⁸ cells/ml solution.

The results also show that the yeast increased dwarf tomato biomass when the seeds were grown on filter paper treated with 10⁸ cells/ml yeast solution (Figure 2). Dwarf tomato germination had improved at the first measurement time (assessment 1) after four days in yeast-treated plants with the following treatments: seeds were soaked in 10⁸ and 10⁹ cells/ml yeast solution or grown on filter paper treated with 10⁸ and 10⁹ cells/ml yeast solution (Figure 3). In addition, yeast metabolites had a positive effect on germination after four days in 10⁸ cells/ml filter paper treatments and 10⁹ cells/ml soaking treatments.

EXAMPLE 3. Investigating the inhibition effect of yeast and yeast metabolites against diseases on *Fusarium graminearum* infected seeds.

Procedure: The trial was performed in Petri dishes on wheat (variety Premio). Seeds were sanitized, rinsed and placed in Petri dishes (5 seeds/Petri dish) on a filter paper. The trial was performed using the Neova yeast and a heat-treated yeast suspension (65 °C for 10 min) in order to evaluate the potential of the yeast strain metabolites. Samples were prepared by soaking the seeds or by wetting the filter paper (2ml/Petri dish) in/with a suspension. Samples were prepared with cell concentration 10⁸ and 10⁹ per ml. The trial was organized in 6 independent replicates of 1 Petri dish per modality. Each Petri dish contained 5 seeds. Evaluated modalities were compared to control modality treated with water (2ml/Petri dish). The extent of fungal infection in plants was determined visually after 10 days.

### Results

Soaking the wheat seeds with yeast solution (both 10⁸ and 10⁹ cells per ml densities) statistically significantly reduced wheat *Fusarium* symptoms and yeast metabolic products (both 10⁸ and 10⁹ cells per ml densities) also had a reducing effect on the symptoms of Fusarium fungal disease (Figure 4).

### EXAMPLE 4. Antifungal activity of living Neova yeast

### Materials and methods

The aim of the experiment was to measure the antifungal activity of the Neova yeast strain. Neova yeast strain was grown on PDA (Sigma) medium in the dark at room temperature for two days. The antifungal activity of yeast was tested against *Moliniafructicola, Botrytis cinerea, Sclerotinia sclerotiorum* and *Fusarium graminearum* that had grown on PDA plates in the dark at room temperature for ten days. Antifungal activity was performed on Petri dish containing PDA medium. Small amount of yeast was transferred on the other side of the Petri dish and fungal isolate was transferred on the opposite side. Control plates contained *F*. *graminearum, S. sclerotiorum, B. cinerea* and *M. fructicola* only. Plates were kept in the dark at room temperature for 5 days for *S. sclerotiorum, F. graminearum* and *B. cinerea* and two months for *M. fructicola* after which fungal growth was measured or pictured.

### Results

Neova yeast strain had antifungal activity against *F*. *graminearum, S. sclerotiorum, B. cinerea* and *M. fructicola.* After five days of incubation, the growth of *F*. *graminearum, S. sclerotiorum* and *B. Cinerea* was clearly reduced on the plates supplemented with yeast when compared to control plates (Figure 5). After two months of incubation, NeHi clearly reduced the growth of *M*. *fructicola* (Figure 6).

### EXAMPLE 5. Neova yeast and its plant growth promoting activity on barley

### Materials and methods

For the first experiment Neova yeast strain was grown in 100 ml of LB broth (Sigma) at 28°C in a rotary shaker (200 rpm min⁻¹) over night and on the following day barley seeds were inoculated with yeast in LB broth, LB broth and water for 8 hours. Germination and plant growth promoting test was performed with 100 seeds for each treatment. In brief, after each of the treatments, barley seeds were placed on a wetted germination paper (20 seeds/paper) and each paper was rolled and wrapped in plastic. Seeds were kept in a growth cabinet (MLR-350, Sanyo Electric Co., Ltd., Gunma, Japan). After seven days, the weight of barley stem and root was measured. Concentration of yeast culture was 830 000 cfu/ml.

For the second experiment two liquid culture of Neova yeast strain were grown overnight in 100 ml of LB medium (Sigma) at 28°C in a rotary shaker (200 rpm min⁻¹) over night. On the following day one yeast culture was heat treated at 65 °C for 10 min. Barley seeds were treated with water, liquid LB medium, liquid living yeast suspension and heat-treated yeast suspension for two hours. Germination and plant growth promoting test was performed as described above.

### Results

The results from the first experiment showed that Neova yeast strain induced the growth of barley roots and stem when compared to water treatment (Figure 7).

The second experiment showed that the Neova yeast induced the growth of barley (Figures 8 and 9). When compared to LB treatment, the yeast induced significantly the biomass of barley roots and stem. Heat-treated yeast suspension (killed yeast) did not induce the growth in a similar manner as living yeast.

### REFERENCES

Marttinen E.M., Niemi-Kapee J., Laaka-Lindberg S., Valkonen J.P.T. 2020. Fungal pathogens infecting moss green roofs in Finland. Urban Forestry & Urban Greening 55 (2020) 126812.
Filipowicz N., et al., Enzyme and Microbial Technology 141 (2020) 109663.
Filipowicz N., et al., Water Air Soil Pollut (2017) 228:210.
Golubev W.I., et al., Microbiology, 2014, Vol. 83, No. 5, pp. 661-665.
Haj-Ahmad Y., et al., Canadian Journal of Microbiology, 1992, Vol. 38, No. 11, pp. 1156-1161.

## Claims

1. A plant biostimulant and/or biocontrol composition for improving plant growth or plant development comprising a yeast of genus *Nadsonia* or killed and/or lysed cells of the yeast, wherein said yeast is the yeast strain of *Nadsonia starkeyi-henricii* deposited at the VTT Culture Collection with accession number VTT C-221063.

2. The composition according to claim 1, wherein said yeast comprises the ITR sequence of SEQ ID NO:1.

3. The composition according to claim 1 or 2, wherein said composition comprises the yeast strain deposited at the VTT Culture Collection with accession number VTT C-221063.

4. The composition according to any of claims 1-3, wherein said composition is a dried powder, an aqueous liquid, in a form of granules, or in a form of pellets.

5. The composition according to any of claims 1-4, wherein said yeast is encapsulated in said composition to provide extended-release properties for the composition.

6. A fertilizer comprising the biostimulant composition according to any of claims 1-5.

7. The fertilizer according to claim 6, wherein said biostimulant composition comprises the yeast strain deposited at the VTT Culture Collection with accession number VTT C-221063.

8. A yeast deposited at the VTT Culture Collection with accession number VTT C-221063.

9. A method for improving plant growth or plant development comprising a step of applying to a plant, or a seed thereof, a plant biostimulant composition according to any of claims 1-5.

10. The method according to claim 9, wherein the plant biostimulant composition is applied as a powder, granule, pellet, aqueous liquid, or microencapsulate.

11. The method according to claim 9 or 10, wherein the plant biostimulant composition comprises the yeast strain deposited at the VTT Culture Collection with accession number VTT C-221063.

12. A method for improving soil quality or remediating degraded soil, the method comprising applying to the soil, or to the plants or plant seeds in said soil, an effective amount of a fertilizer according to claim 6 or 7.

13. The method according to claim 12, wherein said fertilizer is according to claim 7.

## Patentansprüche

1. Pflanzenbiostimulans und/oder Biokontrollzusammensetzung zur Verbesserung des Pflanzenwachstums oder der Pflanzenentwicklung, umfassend eine Hefe der Gattung *Nadsonia* oder abgetöteten und/oder lysierten Zellen der Hefe, wobei es sich bei der Hefe um den Hefestamm *Nadsonia starkeyi-henricii* handelt, der in der VTT-Kultursammlung unter der Zugangsnummer VTT C-221063 hinterlegt ist.

2. Zusammensetzung nach Anspruch 1, wobei die Hefe die ITR-Sequenz SEQ ID NO: 1 umfasst.

3. Zusammensetzung nach Anspruch 1 oder 2, wobei die Zusammensetzung den in der VTT-Kultursammlung unter der Zugangsnummer VTT C-221063 hinterlegten Hefestamm umfasst.

4. Zusammensetzung nach einem der Ansprüche 1-3, wobei es sich bei der Zusammensetzung um ein getrocknetes Pulver, eine wässrige Flüssigkeit, Granulat oder Pellets handelt.

5. Zusammensetzung nach einem der Ansprüche 1-4, wobei die Hefe in der Zusammensetzung verkapselt ist, um der Zusammensetzung Eigenschaften einer verzögerten Freisetzung zu verleihen.

6. Düngemittel, umfassend die Biostimulanszusammensetzung nach einem der Ansprüche 1-5.

7. Düngemittel nach Anspruch 6, wobei die Biostimulanszusammensetzung den in der VTT-Kultursammlung unter der Zugangsnummer VTT C-221063 hinterlegten Hefestamm umfasst.

8. Hefe, die in der VTT-Kultursammlung unter der Zugangsnummer VTT C-221063 hinterlegt ist.

9. Verfahren zur Verbesserung des Pflanzenwachstums oder der Pflanzenentwicklung, umfassend den Schritt des Aufbringens einer Pflanzenbiostimulanszusammensetzung nach einem der Ansprüche 1-5 auf eine Pflanze oder einen Samen derselben.

10. Verfahren nach Anspruch 9, wobei die Pflanzenbiostimulansuusammensetzung als Pulver, Granulat, Pellet, wässrige Flüssigkeit oder Mikroverkapselung aufgebracht wird.

11. Verfahren nach Anspruch 9 oder 10, wobei die Pflanzenbiostimulanszusammensetzung den in der VTT-Kultursammlung unter der Zugangsnummer VTT C-221063 hinterlegten Hefestamm umfasst.

12. Verfahren zur Verbesserung der Bodenqualität oder zur Sanierung degradierter Böden, wobei das Verfahren das Ausbringen einer wirksamen Menge eines Düngemittels nach Anspruch 6 oder 7 auf den Boden oder auf die Pflanzen oder Pflanzensamen in diesem Boden umfasst.

13. Verfahren nach Anspruch 12, wobei es sich bei dem Düngemittel um ein Düngemittel nach Anspruch 7 handelt.

## Revendications

1. Composition de biostimulant et/ou de biocontrôle de plante pour améliorer la croissance de plantes ou le développement de plantes, comprenant une levure du genre *Nadsonia* ou des cellules tuées et/ou lysées de la levure, dans laquelle ladite levure est la souche de levure *Nadsonia starkeyi-henricii* déposée à la collection de cultures VTT sous le numéro d'enregistrement VTT C-221063.

2. Composition selon la revendication 1, dans laquelle ladite levure comprend la séquence ITR de SEQ ID NO: 1.

3. Composition selon la revendication 1 ou 2, dans laquelle ladite composition comprend la souche de levure déposée à la collection de cultures VTT sous le numéro d'enregistrement VTT C-221063.

4. Composition selon l'une quelconque des revendications 1-3, dans laquelle ladite composition est une poudre sèche, un liquide aqueux, sous forme de granulés ou sous forme de pastilles.

5. Composition selon l'une quelconque des revendications 1-4, dans laquelle ladite levure est encapsulée dans ladite composition pour fournir des propriétés de libération prolongée à la composition.

6. Engrais comprenant la composition de biostimulant selon l'une quelconque des revendications 1-5.

7. Engrais selon la revendication 6, dans lequel ladite composition de biostimulant comprend la souche de levure déposée à la collection de cultures VTT sous le numéro d'enregistrement VTT C-221063.

8. Levure déposée à la collection de cultures VTT sous le numéro d'enregistrement VTT C-221063.

9. Procédé d'amélioration de la croissance de plantes ou du développement de plantes comprenant une étape d'application à une plante, ou à une graine de celle-ci, d'une composition de biostimulant de plante selon l'une quelconque des revendications 1-5.

10. Procédé selon la revendication 9, dans lequel la composition de biostimulant de plante est appliquée sous la forme d'une poudre, de granulés, de pastilles, de liquide aqueux ou de microencapsulat.

11. Procédé selon la revendication 9 ou 10, dans lequel la composition de biostimulant de plante comprend la souche de levure déposée à la collection de cultures VTT sous le numéro d'enregistrement VTT C-221063.

12. Procédé d'amélioration de la qualité du sol ou de remise en état d'un sol dégradé, le procédé comprenant l'application au sol, ou aux plantes ou aux graines de plantes dans ledit sol, d'une quantité efficace d'engrais selon la revendication 6 ou 7.

13. Procédé selon la revendication 12, dans lequel ledit engrais est selon la revendication 7.
